(19) Europäisches Patentamt
European Patent Office
Office européen des brevets

(11) Publication number: **0 067 915 A1**

(12) # EUROPEAN PATENT APPLICATION

(21) Application number: 81302843.8

(22) Date of filing: 24.06.81

(51) Int. Cl.³: C 07 C 125/065
A 01 N 47/20

(43) Date of publication of application:
29.12.82 Bulletin 82/52

(84) Designated Contracting States:
AT BE FR GB IT NL SE

(71) Applicant: Gulf Oil Corporation
P. O. Box 1166
Pittsburgh Pennsylvania 15230(US)

(72) Inventor: Gibbs, Charles Gaylord
1901 West 72nd Terrace
Shawnee Mission Kansas 66208(US)

(74) Representative: Huskisson, Frank Mackie et al,
Fitzpatricks 48 St. Vincent Street
Glasgow, G2 5TT Scotland(GB)

(54) **4-Chloro-1-methyl-2-butynyl carbanilates and method of combating wild oats.**

(57) New 4-chloro-1-methyl-2-butynyl carbanilates having the structural formula

$$Ar-NH-\overset{\overset{O}{\|}}{CO}-\overset{\overset{CH_3}{|}}{CH}-C\equiv C-CH_2-Cl$$

in which Ar is phenyl, 3-chlorophenyl, 2-fluorophenyl, 3-fluorophenyl or 2,5-difluorophenyl are selective pre-emergent herbicides which are useful in combating wild oats.

EP 0 067 915 A1

# 4-CHLORO-1-METHYL-2-BUTYNYL CARBANILATES AND METHOD OF COMBATING WILD OATS

## Description of the Invention

Wild oats have been selectively combated in the presence of such crops as wheat, barley, rape, flax and sugar beets by applying barban (4-chloro-2-butynyl-m-chlorocarbanilate, U. S. patent 2,906,614) post-emergently when the wild oats are in the two-leaf stage. By employing relatively low application rates when the wild oats are at a sensitive stage of growth, injury to immature crop plants is avoided.

Although barban remains the preferred herbicide for selective wild oat control, it is only a post-emergent herbicide and kills only the oats which have emerged. Wild oats which germinate later in the growing season are unaffected and become a source of re-infestation, season after season.

I have discovered a small group of 4-chloro-1-methyl-2-butynyl carbanilates which have excellent pre-emergent selectivity as wild oat herbicides, as well as substantial post-emergent efficacy. By careful formulation and use, some of these compounds may give both post-emergent and pre-emergent control of wild oats in the presence of grain crops.

Briefly, my method of selectively killing wild oats comprises applying pre-emergently to soil containing wild oat seeds an effective amount of a compound having the structural formula

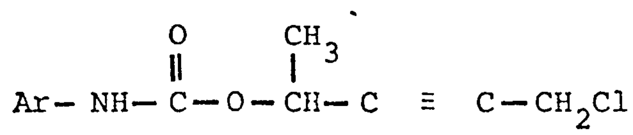

in which Ar is selected from the group consisting of phenyl, 3-chlorophenyl, 2-fluorophenyl, 3-fluorophenyl and 2,5-difluorophenyl. The pronounced pre-emergent selectivity of these compounds is accompanied by substantial post-emergent efficacy. When some of the wild oats have germinated but the wheat, for example, has not yet emerged, a single application will kill the wild oats both post-emergently and pre-emergently. The characteristics of the herbicides in this small group are not shared by compounds of closely related structures. For example, when Ar is 3-bromophenyl, 3-trifluoromethylphenyl, 4-chlorophenyl, 2,6-dichlorophenyl, 2,3-dichlorophenyl, 2,5-dichlorophenyl or 3-ethylphenyl the desirable pre-emergent activity appears to be totally absent, as well as any useful post-emergent activity. When Ar is 4-fluorophenyl, both wild oats and wheat are killed pre-emergently but there is very little effect on other species.

Synthesis of the Herbicides

The novel herbicides employed in the method of this invention may be made from purchased raw materials by means of the procedures presented below for illustrative purposes.

The procedure may be outlined as follows;

$$HC\equiv CCH_2Cl \xrightarrow[\text{THF},\ -40^\circ]{\text{RLi}} Li^{+}\,{}^{-}C\equiv CCH_2 + CH_3CHO \xrightarrow[H^{+}]{\text{THF}}$$

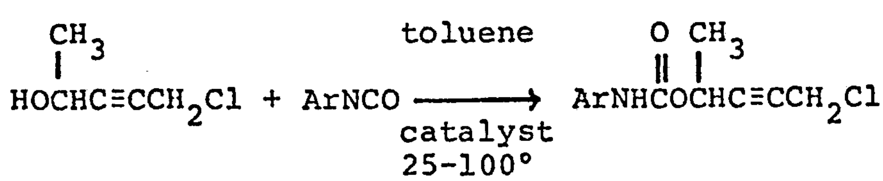

wherein RLi can be any alkyl or aryl lithium reagent such as methyl lithium, ethyl lithium, n-butyl lithium, t-butyl lithium, phenyl lithium and the like. In place of the alkyl or aryl lithium reagents one can substitute alkyl or aryl Grignard reagents for the metalation step. One can use any inert ether in place of tetrahydrofuran (THF) such as diethyl ether, dimethoxyethane, or dioxane. For the arylisocyanate reaction one can use in place of toluene any inert solvent such as benzene, ether, ethyl acetate, dichloromethane and the like, and the catalyst may consist of basic amines, tin salts, and the like. Actual synthesis is exemplified below.

Synthesis of 5-chloro-3-pentyne-2-ol

To a solution of propargyl chloride (11.1 g; 0.15 moles) in 100 ml of dry THF, cooled to -40°C. under an argon atmosphere, was added dropwise 104 ml of a 1.45 M ethereal solution of methyl lithium. After complete addition the mixture was allowed to stir at -40°C. for 15 minutes, then acetaldehyde (6.6g; 0.15 moles) was added dropwise at -40°C. After complete addition the reaction mixture was allowed to come to room temperature and stirred for an additional 1 hr. The reaction mixture was quenched with 10% HCl, evaporated almost to dryness, and taken up in ether. The ether solution was extracted with water and saturated aqueous sodium bicarbonate. The ether layer was dried over anhydrous magnesium sulfate, filtered, and evaporated to dryness. Bulb to bulb distillation gave 9.0 g (51%) of the desired product, b.p. 80-82° at 0.8 mm.

Synthesis of 5-chloro-3-pentynyl-2-N-(3-chlorophenyl)carbamate

To a solution of 5-chloro-3-pentyne-2-ol (1.8 g; 0.015 moles) in 50 ml of dry toluene was added 3-chlorophenylisocyanate (2.3 g; 0.015 moles) and one drop of stannous octoate. The reaction mixture was refluxed for 4 hours and evaporated to dryness to give the desired product as an oil. The IR and NMR spectra were consistent with the proposed structure.

All compounds listed in Table I were prepared by the above procedure.

TABLE I

Compounds of the formula: $Ar{-}NH{-}\overset{\overset{O}{\|}}{C}O{-}\overset{\overset{CH_3}{|}}{C}H{-}C \equiv C{-}CH_2{-}Cl$

| Compound No. | Ar | Method of Identification |
|---|---|---|
| 1 | 3-chlorophenyl | IR,NMR[a] |
| 2 | 3-fluorophenyl | IR,NMR |
| 3 | 2,5-difluorophenyl | IR,NMR |
| 4 | phenyl | IR,NMR |
| 5 | 2-chlorophenyl | IR,NMR |
| 6 | 2-fluorophenyl | IR,NMR |

a. Oils were identified by their infrared and NMR spectra.

Combating Unwanted Vegetation

The novel herbicides of this invention are effective when used post- or pre-emergently. In some instances they may be applied by both techniques. In commercial use the compounds are formulated as herbicidal compositions in combination with an inert diluent and a surface active agent. There is described below an illustrative procedure for herbicidal use of the compounds under controlled conditions in the greenhouse so as to obtain data on phytotoxic activity and selectivity.

Post Emergent Use

An aqueous dispersion of each active compound is prepared by combining 0.4 gram of the compound with about 4 ml of a solvent-emulsifier mixture (3 parts of a commercial polyoxyethylated vegetable oil emulsifier, 1 part xylene, 1 part kerosene) and then adding water, with stirring, to a final volume of 40 ml.

The species of plants on which each compound is to be tested are planted in 4-inch pots in a greenhouse. Ten to 18 days after emergence of the plants, three pots of each species are sprayed with an aqueous dispersion of the active compound prepared as described above, at the indicated rate of active compound per acre and at a spray volume of 60 gallons per acre. Approximately 2 weeks after the spray application the plants are observed and the results rated according to the following schedule:

DEGREE OF EFFECT

0 = no effect
1 = slight effect (temporary injury)
2 = moderate effect (some permanent injury)
3 = severe effect (some plants died)
4 = maximum effect (all plants died)

The same rating schedule is employed to judge pre-emergent results obtained according to the procedure below.

Pre-emergent Use

A solution of each active compound is prepared by dissolving 290 mg of the compound to be tested in 200 ml of acetone. Disposable expanded polystyrene trays about 2 1/2 inches deep and about 1 square foot in area are prepared and sprayed with the acetone solution at the indicated rate of

active chemical per acre of sprayed area and are then covered with about one-fourth inch of soil. Twenty-one days after seeding and treatment the plants are examined and herbicidal effects are rated according to the above schedule.

Both post-emergent and pre-emergent results which have been obtained with the herbicides are set forth in the following tables. Results on a large number of plant species are shown so as to make clear the selective nature of these novel herbicides.

| Compound No: / PLANT SPECIES | Appl'n. Rate (lb./A.) | 1 Pre | 1 Post | 2 Pre | 2 Post | 3 Pre | 3 Post | 4 Pre | 4 Post | 5 Pre | 5 Post | 6 Pre | 6 Post |
|---|---|---|---|---|---|---|---|---|---|---|---|---|---|
| *Xanthium pensylvanicum* | 3 | 0 | | 0 | 1 | 0 | 0 | 0 | 0 | 0 | | 0 | 0 |
| Cocklebur | 1 | 0 | 0 | 0 | 0 | 0 | 0 | 0 | 0 | 0 | | 0 | 0 |
| *Chenopodium album* | 3 | 4 | | 2 | 4 | 1 | 2 | 3 | 3 | 0 | | 0 | 1 |
| Lambsquarters | 1 | 1 | 3 | 0 | 1 | 0 | | 0 | 4 | 0 | | 0 | 0 |
| *Ipomoea purpurea* | 3 | 0 | | 0 | 1 | 0 | 0 | 0 | 3 | 0 | | 0 | 1 |
| Morning glory | 1 | 0 | 0 | 0 | 0 | 0 | 0 | 0 | 1 | 0 | | 0 | 0 |
| *Amaranthus retroflexus* | 3 | 4 | | 4 | 3 | 4 | 3 | 4 | 2 | 0 | | 1 | 1 |
| Pigweed | 1 | 1 | | 1 | 2 | 3 | 1 | 0 | 2 | 0 | | 0 | 0 |
| | 3 | 0 | | 2 | 3 | 3 | 3 | 3 | 3 | 1 | | 2 | 3 |
| Wild buckwheat | 1 | 1 | 3 | 0 | 3 | 1 | 1 | 3 | 2 | 0 | | 0 | 1 |
| *Brassica kaber* | 3 | 3 | | 0 | 3 | 1 | 2 | 4 | 2 | 0 | | 1 | 1 |
| Wild Mustard | 1 | 0 | 4 | 0 | 2 | 0 | 0 | 4 | 0 | | | 0 | 0 |
| *Echinochloa crusgalli* | 3 | 1 | | 1 | 3 | 1 | 2 | 0 | 0 | 0 | | 0 | 0 |
| Barnyard grass | 1 | 0 | 1 | 0 | 1 | 0 | 0 | 0 | 0 | 0 | | 0 | 0 |
| *Digitaria sanguinalis* | 3 | 4 | | 4 | 3 | 3 | 3 | 2 | 1 | 0 | | 0 | 0 |
| Crabgrass | 1 | 2 | 4 | 2 | 1 | 1 | 1 | 0 | 0 | 0 | | 0 | 0 |
| *Bromus tectorum* | 3 | 0 | | 1 | 4 | 3 | 4 | 0 | 3 | 0 | | 0 | 0 |
| Downy Brome | 1 | 0 | 3 | 0 | 4 | 0 | 3 | 0 | 0 | 0 | | 0 | 0 |
| *Setaria faberii* | 3 | 4 | | 4 | 3 | 3 | 3 | 1 | 3 | 0 | | 0 | 0 |
| Giant Foxtail | 1 | 2 | 3 | 2 | 1 | 1 | 1 | 0 | 1 | 0 | | 0 | 0 |
| *Setaria viridis* | 3 | 4 | | 4 | 4 | 2 | 4 | 3 | 3 | 2 | | 0 | 1 |
| Green Foxtail | 1 | 1 | 4 | 1 | 3 | 1 | 2 | 0 | 1 | 0 | | 0 | 0 |
| *Cyperus esculentis* | 3 | 0 | | 0 | 0 | 0 | 0 | 0 | 0 | 0 | | 0 | 0 |
| Nutsedge | 1 | 0 | 0 | 0 | 0 | 0 | 0 | 0 | 0 | 0 | | 0 | 0 |
| *Sorghum bicolor* | 3 | 1 | | 1 | 4 | 0 | 3 | 0 | 1 | 0 | | 0 | 0 |
| Shatter Cane | 1 | 1 | 2 | 0 | 3 | 0 | 3 | 0 | 0 | 0 | | 0 | 0 |
| *Avena fatua* | 3 | 4 | | 3 | 4 | 4 | 4 | 4 | 4 | 4 | | 4 | 4 |
| Wild Oats | 1 | 2 | 4 | 1 | 4 | 3 | 3 | 3 | 4 | 1 | | 0 | 4 |
| *Medicago sativa* | 3 | 2 | | 0 | 1 | 0 | 1 | 3 | 0 | 0 | | 0 | 1 |
| Alfalfa | 1 | 0 | 1 | 0 | 0 | 0 | 0 | 0 | 0 | 0 | | 0 | 0 |
| *Gossypium herbaceum* | 3 | 1 | | 0 | 3 | 1 | 2 | 0 | 0 | 0 | | 0 | 0 |
| Cotton | 1 | 0 | | 0 | 1 | 0 | 1 | 0 | 0 | 0 | | 0 | 0 |
| *Arachis hypogaea* | 3 | 0 | | 0 | 1 | 0 | 0 | 0 | 0 | 0 | | 0 | 0 |
| Peanut | 1 | 0 | 0 | 0 | 0 | 0 | 0 | 0 | 0 | 0 | | 0 | 0 |
| *Soja max* | 3 | 0 | | 0 | 1 | 0 | 1 | 0 | 1 | 0 | | 0 | 1 |
| Soybean | 1 | 0 | 1 | 0 | 0 | 0 | 0 | 0 | 1 | 0 | | 0 | 0 |
| *Beta vulgaris* | 3 | 0 | | 1 | 2 | 1 | 2 | 1 | 2 | 0 | | 0 | 2 |
| Sugar Beets | 1 | 0 | 2 | 0 | 2 | 0 | 1 | 0 | 1 | 0 | | 0 | 0 |
| *Lycopersicum esculentum* | 3 | 3 | | 1 | 3 | 1 | 3 | 3 | 3 | 0 | | 1 | 2 |
| Tomato | 1 | 3 | 3 | 0 | 3 | 0 | 2 | 0 | 2 | 0 | | 0 | 0 |
| *Zea mays* | 3 | 1 | | 1 | 3 | 0 | 3 | 0 | 4 | 0 | | 0 | 0 |
| Corn | 1 | 0 | 3 | 0 | 3 | 0 | 3 | 0 | 2 | 0 | | 0 | 0 |
| *Sorghum vulgare* | 3 | 1 | | 1 | 3 | 0 | 3 | 0 | 0 | 0 | | 0 | 0 |
| Grain Sorghum | 1 | 1 | 2 | 0 | 3 | 0 | 3 | 0 | 0 | 0 | | 0 | 0 |
| *Oryza sativa* | 3 | 0 | | 1 | 3 | 1 | 2 | 1 | 1 | 0 | | 0 | 1 |
| Rice | 1 | 0 | 1 | 0 | 1 | 0 | 0 | 0 | 0 | 0 | | 0 | 0 |
| *Triticum aestivum* | 3 | 0 | | 0 | 4 | 1 | 4 | 1 | 4 | 0 | | 0 | 4 |
| Wheat | 1 | 0 | 4 | 0 | 3 | 0 | 2 | 0 | 4 | 0 | | 0 | 1 |

By examination of the tabulated results it can be seen that the novel herbicides are effective against wild oats when applied pre-emergently at rates of 3 pounds per acre or less, without causing permanent injury to wheat. If the wheat has not yet emerged at the time of application, it will also be seen that wild oats which have already emerged will also be killed, without injury to wheat. In this way substantial control of wild oats can be obtained with a single application of only one herbicide. It is believed that repeated use of the method of this invention, year after year will cause a reduction of wild oat infestations, whereas the presently used methods have been unable to prevent reinfestation during every growing season.

Claims:

1. A method of selectively killing wild oats comprising the step of applying pre-emergently to soil containing wild oat seeds an effective amount up to 3 pounds per acre, of a compound having the structural formula:

$$Ar-NH-\overset{O}{\overset{\|}{C}}-O-\overset{CH_3}{\overset{|}{C}H}-C \equiv C-CH_2Cl$$

in which Ar is selected from the group consisting of phenyl, 2-chlorophenyl, 3-chlorophenyl, 2-fluorophenyl, 3-fluorophenyl and 2,5-difluorophenyl.

2. A method as claimed in claim 1, in which Ar is phenyl.

3. A method as claimed in claim 1, in which Ar is 3-chlorophenyl.

4. A method as claimed in claim 1, in which Ar is 3-fluorophenyl.

5. A method as claimed in claim 1, in which Ar is 2-fluorophenyl.

6. A method as claimed in claim 1, in which Ar is 2,5-difluorophenyl.

7. 4-Chloro-1-methyl-2-butynyl carbanilate.

8. 4-Chloro-1-methyl-2-butynyl N-(3-chlorophenyl)-carbamate.

9. 4-Chloro-1-methyl-2-butynyl N-(3-fluorophenyl)-carbamate.

10. 4-Chloro-1-methyl-2-butynyl N-(2-fluorophenyl)-carbamate.

11. 4-Chloro-1-methyl-2-butynyl N-(2,5-difluorophenyl)-carbamate.

12. 4-Chloro-1-methyl-2-butynyl N-(2-chlorophenyl) carbamate.

Claims:

1. A method of selectively killing wild oats comprising the step of applying pre-emergently to soil containing wild oat seeds an effective amount up to 3 pounds per acre, of a compound having the structural formula:

$$Ar-NH-\overset{\overset{O}{\parallel}}{C}-O-\overset{\overset{CH_3}{|}}{C}H-C\equiv C-CH_2Cl$$

in which Ar is selected from the group consisting of phenyl, 2-chlorophenyl, 3-chlorophenyl, 2-fluorophenyl, 3-fluorophenyl and 2,5-difluorophenyl.

2. A method as claimed in claim 1, in which Ar is phenyl.

3. A method as claimed in claim 1, in which Ar is 3-chlorophenyl.

4. A method as claimed in claim 1, in which Ar is 3-fluorophenyl.

5. A method as claimed in claim 1, in which Ar is 2-fluorophenyl.

6. A method as claimed in claim 1, in which Ar is 2,5-difluorophenyl.

0067915

European Patent Office

# EUROPEAN SEARCH REPORT

Application number
EP 81 30 2843

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | US - A - 3 203 949 (HOPKINS AND PULLEN)<br>* claims, description * | 1-12 | C 07 C 125/065<br>A 01 N 47/20 |
| | --------- | | |

TECHNICAL FIELDS SEARCHED (Int.Cl. 3)

C 07 C 125/00

CATEGORY OF CITED DOCUMENTS

X: particularly relevant if taken alone
Y: particularly relevant if combined with another document of the same category
A: technological background
O: non-written disclosure
P: intermediate document
T: theory or principle underlying the invention
E: earlier patent document, but published on, or after the filing date
D: document cited in the application
L: document cited for other reasons

&: member of the same patent family, corresponding document

The present search report has been drawn up for all claims

| Place of search | Date of completion of the search | Examiner |
|---|---|---|
| The Hague | 18-02-1982 | GAUTIER |

EPO Form 1503.1 06.78